# FASCICULE DE BREVET EUROPEEN

(11) **EP 3 389 574 B1**
(45) Date de publication et mention de la délivrance du brevet: **05.02.2020**
(21) Numéro de dépôt: 16829409.8
(22) Date de dépôt: 10.12.2016
(51) Int. Cl.: A61F 5/02

(54) **CEINTURE DE TRANSPORT ET DE MAINTIEN D'UN DISPOSITIF MEDICAL ET KIT CORRESPONDANT**
RIEMEN ZUM TRANSPORTIEREN EINER MEDIZINISCHEN VORRICHTUNG UND ZUR BEWAHRUNG AN EINEM ORT SOWIE ENTSPRECHENDES KIT
BELT FOR TRANSPORTING A MEDICAL DEVICE AND MAINTINING SAME IN PLACE, AND CORRESPONDING KIT

(30) Priorité: 16.12.2015 FR 1562552
(43) Date de publication de la demande: 24.10.2018
(73) Titulaire: Oxsitis, 63119 Chateaugay (FR)
(72) Inventeur: ANDRIEUX, Fabien, 63200 Riom (FR)
(74) Mandataire: Dennemeyer & Associates S.A.
(86) Numéro de dépôt international: PCT/FR2016/053323
(87) Numéro de publication internationale: WO 2017/103400

(56) Documents cités:
- EP-A1- 0 075 753
- US-A- 5 665 057
- US-A1- 2009 054 844
- US-A1- 2009 089 913
- US-A1- 2013 261 523

## Description

La présente invention concerne une ceinture de transport et de maintien d'un dispositif médical ainsi qu'un kit correspondant.

Pour plusieurs pathologies, il est nécessaire que le patient ait un traitement permanent, jour et nuit, sans interruption. Dans de tels cas, il s'avère que le patient soit doit prendre en permanence un médicament soit doit porter en permanence un dispositif médical, cela que le dispositif comprenne ou non une partie implantée dans le corps du patient.

Lorsque le patient est hospitalisé, ou pour le moins alité, il est relativement aisé de mettre en œuvre un tel traitement, du fait de l'absence, ou du moins du peu de déplacement du patient. En revanche, lorsque l'état général du patient est satisfaisant et que ce dernier peut avoir une vie sociale et/ou professionnelle le plus proche possible de la normale, la question de la mobilité du moyen de distribution du médicament et/ou du dispositif médical se pose.

En particulier, il est fréquent que le patient soit obligé de se déplacer avec un réceptacle de stockage et/ou de distribution d'un médicament et/ou un dispositif médical et/ou une source d'énergie pour un dispositif médical. Pour maintenir le dispositif ou le réceptacle sur le patient en limitant autant que possible la gêne occasionnée par ce transport on connait diverses solutions. Parmi celles-ci ont peut citer celles divulguées par WO-A-2009 045 532 qui concerne une ceinture sur laquelle est fixée une poche. On connait aussi par EP-A-1 514 571 une ceinture formée par une bande auto-agrippante qui maintient en position un dispositif d'injection d'un traitement comportant également un réservoir monté sur un chariot mobile ou bien encore par WO-A-9715252 une ceinture pour transporter un sac de colostomie. On connait également par EP-A-75753 un harnais comportant deux parties dorsale et ventrale réglables autour de l'abdomen, ce harnais étant maintenu sur les épaules du patient par deux bretelles.

Ces différentes solutions ne sont pas pleinement satisfaisantes. En effet, le poids et/ou l'encombrement du dispositif médical à transporter génère fréquemment, si ce n'est une fatigue, au moins une gêne pour le patient qui porte de telles ceintures.

C'est à ces inconvénients qu'entend plus particulièrement remédier l'invention en proposant une ceinture de transport et de maintien d'un dispositif médical, aisée à porter, sans fatigue par le patient tout en ne gênant pas ses mouvements.

A cet effet, l'invention a pour objet une ceinture de transport et de maintien d'un dispositif médical comportant au moins un organe de réception dudit dispositif, ladite ceinture comprenant au moins une bande en matériau souple et au moins une sangle de longueur réglable, la bande en matériau souple étant un quadrilatère dont le rapport longueur sur largeur est au moins égal à 1,5, caractérisée en ce qu'elle comprend deux bandes en matériau textile reliées par quatre sangles amovibles.

Ainsi, avec une telle largeur de la ceinture on assure un maintien confortable de celle-ci autour de la taille d'un patient. Le poids du dispositif est réparti sur une surface telle que la traction exercée sur les régions abdominale et lombaire du patient est minimale. Par ailleurs, un tel rapport longueur sur largeur limite les mouvements de déplacement de la ceinture autour de la taille du patient. La présence d'au moins une bande maintenue de manière réglable par au moins une sangle permet d'ajuster de manière optimale et individualisée la tension de la ceinture autour de la taille du patient. En d'autres termes, le confort est accru, tout en assurant un maintien optimal du dispositif.

Selon des aspects avantageux mais non obligatoires de l'invention, une telle ceinture peut comprendre une ou plusieurs des caractéristiques suivantes:
- Les sangles sont pourvues à leurs extrémités libres de zones auto-agrippantes.
- Une encoche ménagée sur un bord d'une bande et un organe de maintien ménagé à proximité de l'autre bord de la bande forment des moyens de maintien d'un câble, conduit ou gaine.
- L'organe de réception du dispositif médical est réalisé en un matériau rigide.
- L'organe de réception est formé de deux parties de formes complémentaires, une partie formant un couvercle amovible.
- Des passages pour des conduits, câbles ou gaines, sont formés dans les bords des parties.
- Au moins une des bandes est équipée d'au moins un autre organe de réception.
- Au moins un autre organe de réception équipant au moins une des bandes est une poche souple.
- La ceinture comprend au moins une bande réalisée en un matériau souple imperméable.
- La bande en matériau souple imperméable est équipée d'une poche de réception d'un dispositif médical, la poche étant pourvue d'un moyen d'obturation.
- La bande en matériau souple imperméable est équipée d'une poche de réception d'une bande en matériau souple.

L'invention concerne également un kit de transport et de maintien d'un dispositif médical comprenant au moins une ceinture selon une des caractéristiques précédentes, caractérisé en ce qu'il comprend au moins deux organes de réception de deux dispositifs médicaux différents, au moins deux bandes en matériau souple et au moins quatre sangles.

Selon des aspects avantageux mais non obligatoires de l'invention, un tel kit peut comprendre une ou plusieurs des caractéristiques suivantes:
- Il comprend au moins deux bandes en matériau souple de dimensions et/ou de nature différentes et au moins quatre sangles.
- Il comprend au moins une bande en matériau souple imperméable.

L'invention sera mieux comprise et d'autres avantages de celle-ci apparaitront plus clairement à la lecture de la description qui va suivre de plusieurs modes de réalisation de l'invention, donnée à titre d'exemple non limitatif et faite en référence aux dessins suivants dans lesquels:
- La figure 1 est une vue de dessus d'une face d'une première bande de textile et des sangles de liaison, constitutives d'un mode de réalisation de l'invention, en position de pré-montage,
- la figure 2 est une vue de dessus, à la même échelle, d'une face d'une seconde bande de textile constitutive du mode de réalisation de la figure 1,
- la figure 3 est une vue similaire à la figure 2, à la même échelle, l'organe de réception du dispositif médical étant en position ouverte,
- la figure 4 est une vue de dessus, à la même échelle, d'un moyen de transport, en configuration montée, réalisé à partir des bandes illustrées aux figures précédentes et
- la figure 5 est une vue de dessus d'une bande en matériau souple imperméable selon un autre mode de réalisation de l'invention.

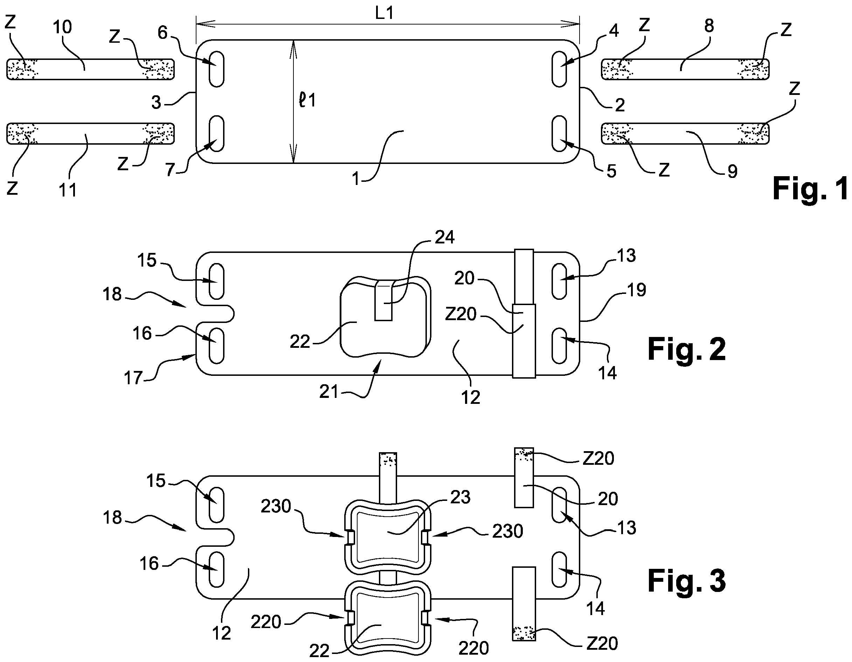

La figure 1 illustre une première bande en matériau souple 1. Ici, la bande 1 est réalisée en textile, en l'espèce en polyester. En variante elle est en polyamide, polyuréthane ou un autre textile synthétique ou naturel. La bande 1 est, dans tous les cas, réalisée en un matériau non seulement souple, mais lavable, non allergique, non inflammable et adapté pour être porté par une personne que ce soit sur un vêtement ou en contact direct avec la peau. La bande 1 est un quadrilatère, donc de forme rectangulaire ou carrée. Avantageusement, elle est de forme rectangulaire.

La bande 1 a une longueur L1 comprise entre 4,5 cm et 40 cm pour une largeur l1 comprise entre 3 cm et 20 cm. Ainsi, on a un rapport L1/I1 au moins égal à 1,5 et avantageusement compris entre 1,5 et 2. Dans tous les cas, la bande 1 doit avoir une largeur l1 minimale de 3 cm. Une telle largeur l1 minimale permet un maintien en position de la bande 1 de textile, sans que celle-ci risque de blesser le porteur de la ceinture et en limitant, si ce n'est en évitant, tout risque de déplacement de la ceinture, par rotation autour de la taille.

Les coins de la bande 1 sont arrondis. Les bords 2, 3 de la bande 1 définissant la largeur l1, sont pourvus, chacun, de deux orifices traversant 4 à 7. Ici, les orifices 4 à 7 sont allongés. En variante non illustrée, ils ont une autre forme et/ou une autre dimension. De même, on conçoit que le nombre d'orifices soit différent, par exemple il est possible de prévoir trois orifices sur chaque bord, si la largeur de la bande l'autorise. Dans tous les cas, les orifices 4 à 7 sont adaptés pour assurer le passage, à jeu minimal, d'au moins une sangle plate 8 à 11. Ici, il est avantageusement prévu quatre sangles 8 à 11.

Chaque sangle 8 à 11 est munie, à ses extrémités libres, d'un moyen de fixation de la sangle à une extrémité d'une autre sangle 11 à 8 ou à l'autre extrémité libre de la même sangle. En l'espèce le moyen de fixation est une zone auto-agrippante Z ménagée aux extrémités des sangles 8 à 11. On conçoit aisément que d'autres moyens de fixation sont utilisables, par exemple, des boutons pression, des fermetures à glissières, des aimants ou autre, pour autant que ces moyens soient manœuvrables rapidement par le porteur de la ceinture.

La figure 2 illustre une seconde bande en matériau souple 12. Ici la nature du matériau souple est également un textile. La forme et les dimensions de la bande 12 sont similaires à celles de la bande 1. En variante, elles sont différentes. En d'autres termes, la bande 12 est rectangulaire. Comme la première bande 1, la bande 12 est pourvue de quatre orifices traversant 13 à 16. Ces orifices 13 à 16 sont similaires aux orifices 4 à 7. En variante, ils sont de forme et/ou de dimensions différentes. Les orifices 13 à 16 sont ménagés sur les bords 17, 19 de la bande 12. Ils permettent également le passage des sangles 8 à 11.

Un bord 17, ici à gauche de la figure 2, définissant la largeur de la bande 12 est pourvu, sensiblement en position médiane, d'une encoche 18. Cette encoche 18 a pour fonction de faciliter le passage de conduits et/ou de câbles lorsque ces derniers doivent passer entre la ceinture et la peau du patient, par exemple lorsqu'ils sont reliés à un dispositif médical implantable. En variante, la disposition et/ou le nombre d'encoches 18 sont différents, étant entendu que des encoches 18 peuvent être prévues sur les deux bords 17, 19. Afin de préserver l'intégrité des câbles et conduits, les bords de l'encoche 18 sont arrondis et équipés, avantageusement, d'un rembourrage.

Le bord 19, opposé et parallèle au bord 17, est équipé, d'un organe de maintien 20 d'un conduit ou d'un câble, non illustrés. L'organe 20 est situé au voisinage des orifices 13, 14. Ici, l'organe 20 est formé par une sangle pourvue d'extrémités libres Z20 auto-agrippantes, permettant ainsi de réaliser une boucle. En variante, la fermeture en boucle de la sangle 20 est obtenue par d'autres moyens connus en soi, par exemple par des boutons pression, des aimants ou une fermeture à glissière.

Sensiblement en position médiane, la bande 12 est équipé d'un organe de réception 21 d'un dispositif médical ou pour le moins d'une partie de ce dernier. Par dispositif médical, on désigne, par exemple, un boitier de commande et/ou d'enregistrement de données physiologiques, une seringue, une batterie, une pompe, un récipient, un moyen de stockage et/ou de distribution d'un médicament ou un autre organe constitutif d'un dispositif médical voire un dispositif médical complet, étant entendu qu'une partie d'un tel dispositif est généralement, mais non obligatoirement, implantée dans le corps du patient, par exemple par voie veineuse. Dans tous les cas, l'usage du dispositif nécessite le maintien sur le corps du patient d'au moins une partie du dispositif, cela de manière si ce n'est permanente ou moins pendant une durée de plusieurs heures consécutives.

L'organe de réception 21 est réalisé en un matériau rigide, adapté à un contact avec un dispositif médical, donc au minimum neutre vis-à-vis de ce dernier, en particulier du point de vue électromagnétique. Avantageusement, l'organe 21 est aisé à nettoyer et à désinfecter. L'organe 21 est également adapté pour être porté par un patient. L'organe 21 se présente sous la forme d'un boitier rigide en deux parties 22, 23. Une partie 22 forme un couvercle de forme complémentaire à l'autre partie 23 dite de réception.

Cette partie 23 a une forme complémentaire de celle du dispositif, de sorte à recevoir le dispositif avec un jeu minimal, évitant tout mouvement du dispositif lorsqu'il est en place dans la partie 23. En variante non illustrée, des organes de calage tels que des blocs en mousse, sont prévus.

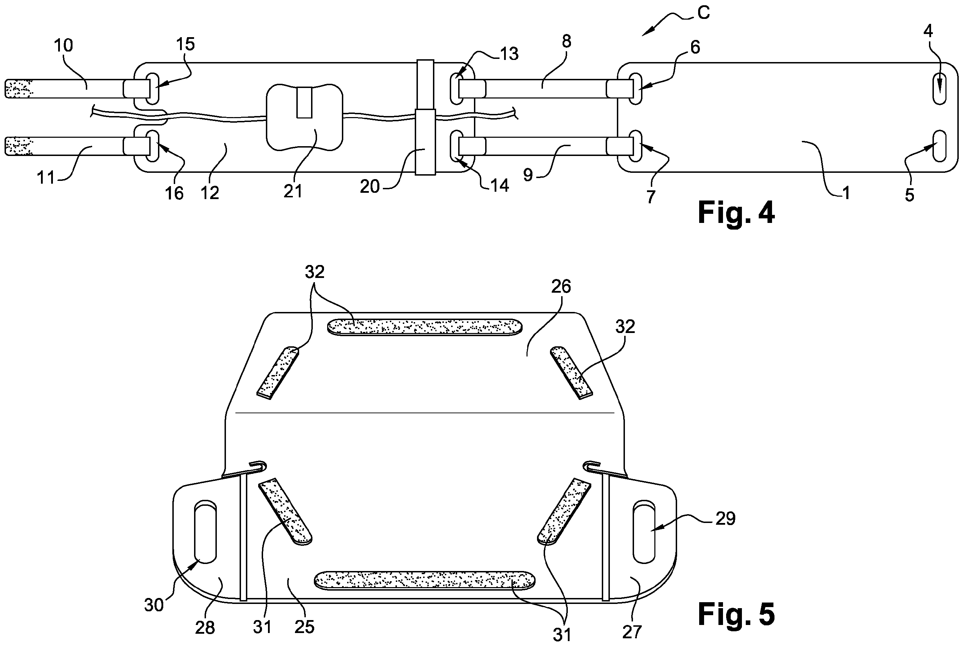

Lorsque le couvercle 22 est en position sur la partie 23 de réception, comme illustré aux figures 2 et 4, le volume défini par les parties 22, 23, donc de facto le volume interne utile de l'organe 21, est fermé. Ainsi, on préserve le dispositif médical de la poussière et/ou des projections d'eau tout en permettant, si besoin, la dissipation de la chaleur générée par le fonctionnement du dispositif et/ou la communication, sans fil, du dispositif avec un organe de commande ou de collecte de données distant de l'organe 21.

On conçoit que, en variante, un joint d'étanchéité soit prévu entre les parties 22 et 23 afin d'améliorer la protection contre la projection d'eau. Un tel joint peut être remplacé par une configuration géométrique du couvercle 22 qui recouvre partiellement, la partie 23, limitant ainsi toute entrée d'eau dans le volume de l'organe 21. Dans un autre mode de réalisation non illustré, une housse amovible permet de recouvrir l'organe 21 et de protéger le dispositif médical contre toute entrée d'eau.

Des encoches 220, 230, avantageusement aux bords recouverts d'un matériau souple, par exemple un élastomère, sont ménagées dans au moins une des parties 22, 23 afin de permettre le passage de câbles, gaines et/ou conduits reliant le dispositif au corps du patient. Ici, chaque partie 22, 23 est pourvue de deux encoches 220, 230 identiques, en regard l'une de l'autre sur des bords opposés des parties 22, 23. Selon le mode de réalisation avantageux illustré, les encoches 220, 230 de passage pour les câbles, gaines et conduits sont formées par des zones concaves ménagées dans les parties 22, 23. Ces zones ne sont pas jointives lorsque les parties 22, 23 sont en configuration fermée, comme représenté aux figures 2 et 4.

Les parties 22, 23 sont maintenues en configuration fermée par un organe de fermeture 24. Cet organe 24 est aisé à manipuler, insensiblement à l'eau et léger. Avantageusement, il est formé par une bande auto-agrippante.

Comme illustré à la figure 3, lorsque l'on souhaite mettre en place un dispositif médical, non illustré pour plus de lisibilité, dans l'organe 21, outre de configurer en position ouverte les parties 22, 23, il convient de dégager la bande 20 afin de passer les câbles, gaines et/ou conduits sur la bande de textile 12. Cela peut se faire avant de mettre la ceinture autour de la taille du patient ou, en variante, une fois la ceinture en place autour de la taille du patient. Dans tous les cas, il est avantageux de mettre en place le dispositif médical dans l'organe 21 lorsque les bandes 1, 12 sont reliées entre elles par les sangles 8 à 11 pour former la ceinture C.

Comme cela ressort à la figure 3, pour réaliser la ceinture C, on réunit les bandes en matériau souple, dans l'exemple deux bandes de textile 1, 12, par les sangles, ici quatre sangles 8 à 11, introduites dans les orifices 4 à 7. Il convient de permettre à la ceinture C de faire le tour de la taille du patient sans serrer, tout en autorisant les mouvements du patient et en maintenant la ceinture C en place, c'est-à-dire sans que celle-ci effectue des mouvements de rotation ou de déplacement vertical autour de la taille du patient lorsque ce dernier bouge. A cet effet, il est aisé de régler précisément et autant de fois que nécessaire la longueur des sangles 4 à 7 une fois la ceinture C en place, par exemple en fonction des vêtements portés par le patient.

La largeur de chaque bande textile 1, 12 constitutive de la ceinture C permet une répartition régulière de la pression exercée par la tension de la ceinture et du poids du dispositif sur une surface suffisante pour que le port quotidien et permanent de la ceinture C ne soit pas contraignant. Par ailleurs, une telle configuration, en au moins une et de préférence deux bandes en matériau souple relativement larges par rapport à leurs longueurs, permet, alliée avantageusement à la nature du matériau souple, ici un textile, d'éviter ou pour le moins de limiter tout phénomène d'échauffement. La présence de zones à mailles ouvertes dans les bandes participe à limiter également l'apparition d'échauffement ou de réactions allergiques, en facilitant entres autres l'évacuation de la transpiration lorsque la ceinture C est portée directement sur la peau.
Une telle ceinture C permet le maintien d'un dispositif pouvant peser jusqu'à 1000 grammes, sachant que le poids de la ceinture est compris entre 70 grammes et 300 grammes.

Dans un autre mode de réalisation non représenté, l'organe 21 est fixé de manière amovible, par exemple par une bande auto-agrippante, sur la bande de textile 12. En variante, la fixation est réalisée par un autre moyen connu en soi, tel que des boutons pression, une fermeture à glissières, une liaison magnétique ou autre.

La figure 5 illustre un autre mode de réalisation de l'invention dans lequel une bande 25 en un matériau souple imperméable est illustrée. Ici, la bande 25 est en polyuréthane type TPU transparent et biocompatible. En variante elle est en PVC ou en un autre polymère biocompatible et imperméable. La bande 25 comprend un volume central obturable, de manière amovible, par un rabat 26.

En d'autres termes, la bande 25 comporte une poche de réception soit d'un dispositif soit, comme dans l'exemple illustré, d'une bande 1 ou 12. Les bords 27, 28 de la bande 25 sont pourvus d'orifices respectivement 29, 30 de passage des sangles 8 à 11. Des bandes auto-agrippantes 31 fixées sur la bande 25 assurent le maintien en position fermée du rabat 26 par coopération avec des bandes auto-agrippantes 32 complémentaires fixées sur le rabat 26.

On conçoit que, en variante, une telle bande en matériau imperméable est configurée en manchon, par exemple ouvert aux extrémités, propre à recevoir la bande 1 équipée du dispositif 21. En variante, le volume interne de la bande 25 est adapté pour recevoir le dispositif 21. Dans tous les cas, une telle bande 25 permet au patient de transporter le dispositif médical lorsqu'il doit se trouver dans un environnement humide, par exemple lors d'une sortie sous la pluie ou lors d'une douche.

Il est ainsi possible de proposer un kit comportant au moins une bande en matériau souple 1 ; 12, au moins une sangle 8 à 11 et au moins deux organes de réception 21 de deux dispositifs médicaux différents. Les organes 21 qui composent le kit ont des formes et/ou des dimensions adaptées aux dispositifs à maintenir. Un tel kit peut avantageusement comporter des bandes en matériau souple en nombre et/ou en dimensions et/ou en nature de matériau, en particulier la largeur, différents de celles décrites. En particulier, selon un mode de réalisation préféré, le kit comprend deux bandes en matériau souple, au moins deux et avantageusement quatre, sangles et une bande en matériau souple imperméable. En d'autres termes, un tel kit permet de réaliser, à façon et de manière non permanente, une ceinture C particulièrement aisée à adapter au dispositif à recevoir et/ou au patient devant porter la ceinture C.

En variante, les sangles 8 à 11 constitutives sont réalisées en un matériau élastique. De même, les bandes 1 ; 12 peuvent être fabriquées, au moins en partie, avec un matériau élastique. L'utilisation de matériau élastique participe à faciliter le réglage de la ceinture C en augmentant la plage de réglage de la longueur de celle-ci.

En variante, l'organe de réception 21 est pourvu d'un moyen de signalisation de la présence et/ou du fonctionnement d'un dispositif médical lorsque ce dernier est logé dans l'organe. A titre d'exemple, on peut citer un marquage visuel de couleur sur une face visible de l'organe en configuration fermée, une LED et/ou l'émission d'un signal sonore en configuration fermée ou ouverte ou tout autre moyen de signalisation.

De même il est possible de prévoir, en variante, des moyens dits détrompeurs permettant à l'utilisateur de n'introduire qu'un dispositif médical donné ou selon une orientation donnée dans l'organe de réception.

Dans un autre mode de réalisation, il est prévu de maintenir plus d'un dispositif ou du moins plus d'un organe constitutif d'un dispositif sur une telle ceinture. Dans ce cas, la bande 12 est adaptée.

Dans un autre mode de réalisation, la ceinture C est équipée d'autres organes de réception, par exemple des poches souples pour contenir un document, des clés ou un bidon.

Dans un autre mode de réalisation, au moins une bande est équipée de panneaux solaires souples, connus en soi, permettant de recharger au moins partiellement un organe moteur du dispositif médical.

On conçoit aisément que l'invention trouve également son application dans le domaine vétérinaire, pour autant que les dimensions et/ou la forme des bandes et sangles soient adaptées.

## Revendications

1. Ceinture (C) de transport et de maintien d'un dispositif médical comportant au moins un organe de réception (21) dudit dispositif, ladite ceinture (C) comprenant au moins une bande en matériau souple (1 ;12 ; 25) et au moins une sangle (8 à 11) de longueur réglable, la bande en matériau souple (1 ;12 ; 25) étant un quadrilatère dont le rapport longueur (L1) sur largeur (l1) est au moins égal à 1,5, **caractérisée en ce qu'**elle comprend deux desdites bandes en matériau souple, construites en matériau textile (1 ;12) et reliées par quatre desdites sangles (8 à 11) amovibles.

2. Ceinture selon la revendication 1, **caractérisée en ce que** les sangles (8 à 11) sont pourvues à leurs extrémités libres de zones auto-agrippantes (Z).

3. Ceinture selon l'une des revendications 1 ou 2, **caractérisée en ce qu'**une encoche (18) ménagée sur un bord (17) d'une bande (12) et un organe de maintien (20) ménagé à proximité de l'autre bord (19) de la bande (12) forment des moyens de maintien d'un câble, conduit ou gaine.

4. Ceinture selon la revendication 1, **caractérisée en ce que** l'organe de réception (21) du dispositif médical est réalisé en un matériau rigide.

5. Ceinture selon la revendication 4, **caractérisée en ce que** l'organe de réception (21) est formé de deux parties (22, 23) de formes complémentaires, une partie (22) formant un couvercle amovible.

6. Ceinture selon la revendication 5, **caractérisée en ce que** des passages (220, 230) pour des conduits, câbles ou gaines, sont formés dans les bords des parties (22, 23).

7. Ceinture selon l'une des revendications précédentes, **caractérisée en ce qu'**au moins une des bandes (1 ; 12 ; 25) est équipée d'au moins un autre organe de réception.

8. Ceinture selon la revendication 7, **caractérisée en ce qu'**au moins un autre organe de réception équipant au moins une des bandes (1 ; 12 ; 25) est une poche souple.

9. Ceinture selon la revendication 1, **caractérisée en ce qu'**elle comprend au moins une bande (25) réalisée en un matériau souple imperméable.

10. Ceinture selon la revendication 9, **caractérisée en ce que** la bande (25) en matériau souple imperméable est équipée d'une poche de réception d'un dispositif médical, la poche étant pourvue d'un moyen d'obturation (26).

11. Ceinture selon la revendication 9, **caractérisée en ce que** la bande (25) en matériau souple imperméable est équipée d'une poche de réception d'une bande (1 ; 12) en matériau souple.

12. Kit de transport et de maintien d'un dispositif médical comprenant au moins une ceinture selon une des revendications précédentes, **caractérisé en ce qu'**il comprend au moins deux organes de réception (21) de deux dispositifs médicaux différents, au moins deux bandes (1 ; 12 ; 25) en matériau souple et au moins quatre sangles (8 à 11).

13. Kit selon la revendication 12, **caractérisé en ce qu'**il comprend au moins deux bandes en matériau souple (1 ; 12) de dimensions et/ou de nature différentes et au moins quatre sangles (8 à 11).

14. Kit selon l'une des revendications 12 ou 13, **caractérisé en ce qu'**il comprend au moins une bande (25) en matériau souple imperméable.

## Patentansprüche

1. Riemen (C) zum Transportieren und Halten einer medizinischen Vorrichtung, umfassend mindestens ein Aufnahmeelement (21) der genannten Vorrichtung, wobei der genannte Riemen (C) mindestens einen Streifen aus flexiblem Material (1; 12; 25), und mindestens einen Gurt (8 bis 11) mit einstellbarer Länge umfasst, wobei der Streifen aus flexiblem Material (1; 12; 25) ein Viereck ist, dessen Verhältnis Länge (L1) zu Breite (l1) mindestens gleich 1,5 ist, **dadurch gekennzeichnet, dass** er zwei der genannten Streifen aus flexiblem Material umfasst, die aus Textilmaterial (1; 12) hergestellt, und durch vier der genannten entfernbaren Gurte (8 bis 11) verbunden sind.

2. Riemen nach Anspruch 1, **dadurch gekennzeichnet, dass** die Gurte (8 bis 11) an ihren freien Enden mit Klettverschlusszonen (Z) versehen sind.

3. Riemen nach einem der Ansprüche 1 oder 2, **dadurch gekennzeichnet, dass** eine an einem Rand (17) eines Streifens (12) vorgesehene Kerbe (18), und ein in der Nähe des anderen Randes (19) des Streifens (12) vorgesehenes Halteelement (20) Mittel zum Halten eines Kabels, Kanals oder einer Hülle bilden.

4. Riemen nach Anspruch 1, **dadurch gekennzeichnet, dass** das Aufnahmeelement (21) der medizinischen Vorrichtung aus einem starren Material besteht.

5. Riemen nach Anspruch 4, **dadurch gekennzeichnet, dass** das Aufnahmeelement (21) aus zwei Teilen (22, 23) komplementärer Formen gebildet ist, wobei ein Teil (22) eine abnehmbare Abdeckung bildet.

6. Riemen nach Anspruch 5, **dadurch gekennzeichnet, dass** in den Rändern der Teile (22, 23) Durchgänge (220, 230) für Leitungen, Kabel oder Hüllen ausgebildet sind.

7. Riemen nach einem der vorhergehenden Ansprüche, **dadurch gekennzeichnet, dass** mindestens einer der Streifen (1; 12; 25) mit mindestens einem weiteren Aufnahmeelement ausgestattet ist.

8. Riemen nach Anspruch 7, **dadurch gekennzeichnet, dass** mindestens ein anderes Aufnahmeelement, das mindestens einen der Streifen (1; 12; 25) ausstattet, eind flexible Tasche ist.

9. Riemen nach Anspruch 1, **dadurch gekennzeichnet, dass** er mindestens einen Streifen (25) aus einem undurchlässigen flexiblen Material umfasst.

10. Riemen nach Anspruch 9, **dadurch gekennzeichnet, dass** der Streifen (25) aus undurchlässigem flexiblem Material mit einer Tasche zur Aufnahme einer medizinischen Vorrichtung ausgestattet ist, wobei die Tasche mit einem Verschlussmittel (26) versehen ist.

11. Riemen nach Anspruch 9, **dadurch gekennzeichnet, dass** der Streifen (25) aus undurchlässigem flexiblem Material mit einer Aufnahmetasche für einen Streifen (1; 12) aus flexiblem Material ausgestattet ist.

12. Kit zum Transportieren und Halten einer medizinischen Vorrichtung, umfassend mindestens einen Riemen nach einem der vorhergehenden Ansprüche, **dadurch gekennzeichnet, dass** es mindestens zwei Aufnahmeelemente (21) von zwei verschiedenen medizinischen Vorrichtungen, mindestens zwei Streifen (1; 12; 25) aus flexiblem Material und mindestens vier Gurte (8 bis 11) umfasst.

13. Kit nach Anspruch 12, **dadurch gekennzeichnet, dass** es mindestens zwei Streifen aus flexiblem Material (1; 12) mit unterschiedlichen Abmessungen und/oder Eigenschaften, und mindestens vier Gurte (8 bis 11) umfasst.

14. Kit nach einem der Ansprüche 12 oder 13, **dadurch gekennzeichnet, dass** es mindestens einen Streifen (25) aus undurchlässigem flexiblem Material umfasst.

## Claims

1. A belt (C) for transporting a medical device and maintaining same in place, including at least one receiving member (21) for receiving said device, said belt (C) comprising at least one strip of flexible material (1;12; 25) and at least one length-adjustable strap (8 to 11), the strip of flexible material (1; 12; 25) being a quadrilateral in which the length (L1) to width (11) ratio is at least equal to 1.5, **characterised in that** it comprises two of said strips of flexible material, which are made of textile material (1; 12) and connected by four of said removable straps (8 to 11).

2. The belt according to claim 1, **characterised in that** the straps (8 to 11) are provided with hook-and-loop areas (Z) at their free ends.

3. The belt according to one of claims 1 or 2, **characterised in that** a notch (18) arranged on one edge (17) of a strip (12) and a holding member (20) arranged near the other edge (19) of the strip (12) form means for maintaining a cable, pipe or sheath in place.

4. The belt according to claim 1, **characterised in that** the receiving member (21) for receiving the medical device is made of a rigid material.

5. The belt according to claim 4, **characterised in that** the receiving member (21) is made up of two portions (22, 23) having complementary shapes, one portion (22) forming a removable cover.

6. The belt according to claim 5, **characterised in that** passages (220, 230) for pipes, cables or sheaths are formed in the edges of the portions (22, 23).

7. The belt according to one of the preceding claims, **characterised in that** at least one of the strips (1; 12; 25) is provided with at least one other receiving member.

8. The belt according to claim 7, **characterised in that** at least one other receiving member provided on at least one of the strips (1; 12; 25) is a flexible pouch.

9. The belt according to claim 1, **characterised in that** it comprises at least one strip (25) made of an impervious flexible material.

10. The belt according to claim 9, **characterised in that** the strip (25) of impervious flexible material is provided with a pouch for receiving a medical device, the pouch being provided with a sealing means (26).

11. The belt according to claim 9, **characterised in that** the strip (25) of impervious flexible material is provided with a pouch for receiving a strip (1; 12) made of flexible material.

12. A kit for transporting a medical device and maintaining same in place, comprising at least one belt according to one of the preceding claims, **characterised in that** it comprises at least two receiving members (21) for receiving two different medical devices, at least two strips (1; 12; 25) made of flexible material and at least four straps (8 to 11).

13. The kit according to claim 12, **characterised in that** it comprises at least two strips of flexible material (1; 12) of different dimensions and/or of different natures, and at least four straps (8 to 11).

14. The kit according to one of claims 12 or 13, **characterised in that** it comprises at least one strip (25) made of impervious flexible material.
